# EUROPEAN PATENT SPECIFICATION

(11) **EP 0 985 397 B1**
(45) Date of publication and mention of the grant of the patent: **12.04.2006**
(21) Application number: 99307135.6
(22) Date of filing: 08.09.1999
(51) Int. Cl.: A61F 13/15, A61F 13/56

(54) **Sanitary napkin**
Damenbinde
Serviette hygiénique

(30) Priority: 11.09.1998 JP 25797898
(43) Date of publication of application: 15.03.2000
(73) Proprietor: UNI-CHARM CORPORATION, Shikokuchuo-shi, Ehime-ken (JP)
(72) Inventor: Mizutani, Satoshi, c/o Technical Center, Mitoyo-gun, Kagawa-ken 769-1602 (JP)
(74) Representative: Fitchett, Stuart Paul

(56) References cited:
- EP-A- 0 768 070
- WO-A-95/17148
- WO-A-97/04730
- US-A- 5 454 802

## Description

This invention relates to a sanitary napkin for absorption and containment body exudates.

Japanese Patent Application (PCT) Disclosure Gazette No. Hei6-502336 discloses a sanitary napkin comprising a liquid-pervious topsheet, a liquid-impervious backsheet, a liquid-absorbent core and a liquid-pervious spacer member. The napkin is formed at transversely opposite side edges thereof with a pair of wings, respectively. The spacer member functions to space the topsheet away from the liquid-absorbent core and thereby to improve a fitting of the napkin to the wearer's body. An upper portion of the spacer member which is relatively flat is at least partially deformed convexly upward as the napkin is compressed by the wearer's femora.

The known napkin is adapted to be fastened to an undergarment by folding the wings back onto a lower surface of a crotch region of the undergarment and fastening the napkin to the lower surface of the crotch region by means of adhesive zones formed on the wings. With the napkin of such an arrangement, the transversely opposite side edges thereof are fixed to the undergarment and the spacer member is deformed convexly upward only when the side edges of the napkin are compressed by the wearer's femora. If the napkin more or less slips down and consequently the wearer's femora can not properly compress the side edges of the napkin, the spacer member is not properly deformed convexly upward and makes it impossible to assure a desired fitting between the upper sheet and the wearer's body.

It is an object of this invention to provide a sanitary napkin improved so that the napkin can be reliably deformed convexly upward without relying on a compressive force of the wearer's femora and thereby to assure a good fitting of the napkin to the wearer's body.

According to this invention, there is provided a sanitary napkin having a basic structure including a liquid-pervious topsheet for defining a body-facing side, a liquid-impervious backsheet for defining an undergarment-facing side and a liquid-absorbent core disposed between the topsheet and the backsheet, and deformable means adapted to deform a transversely middle region of the absorbent core convexly toward the body-facing side and fastening means used to detachably fasten the napkin to the undergarment.

The deformable means includes either an elastic sheet presenting an annular shape transversely of the absorbent core or paired inelastic sheet-like members of the same width placed upon each other, and an elastically stretchable/contractible member extending under tension between the paired sheet-like members transversely of the absorbent core and fixed to transversely opposite side edges of the paired sheet-like members and the fastening means extends on an lower surface of the napkin along a longitudinal center line and has a width corresponding to 5 - 50 % of the width of the absorbent core.

According to still another embodiment of this invention, the napkin is bonded by the fastening means to an upper surface of a second napkin contoured to be larger than the napkin.

Embodiments of the invention will now be described, by way of example only, with reference to the accompanying drawings, in which:
Fig. 1 is a perspective view showing a partially cut away sanitary napkin according to one embodiment of this invention;
Fig. 2 is a sectional view taken along a line II-II in Fig. 1;
Fig. 3 is a sectional view taken along a line III-III in Fig. 1;
Fig. 4 is a view similar to Fig. 2 showing the napkin when its elastic members are in a contracted state;
Fig. 5 is a view similar to Fig. 4 showing another embodiment of this invention;
Fig. 6 is a view similar to Fig. 4 showing still another embodiment of this invention; and
Fig. 7 is a view similar to Fig. 4 illustrating a manner in which the sanitary napkin shown in Fig. 1 is possibly used in combination with an additional napkin.

Details of a sanitary napkin according to this invention will be more fully understood from the description given hereunder with reference to the accompanying drawings.

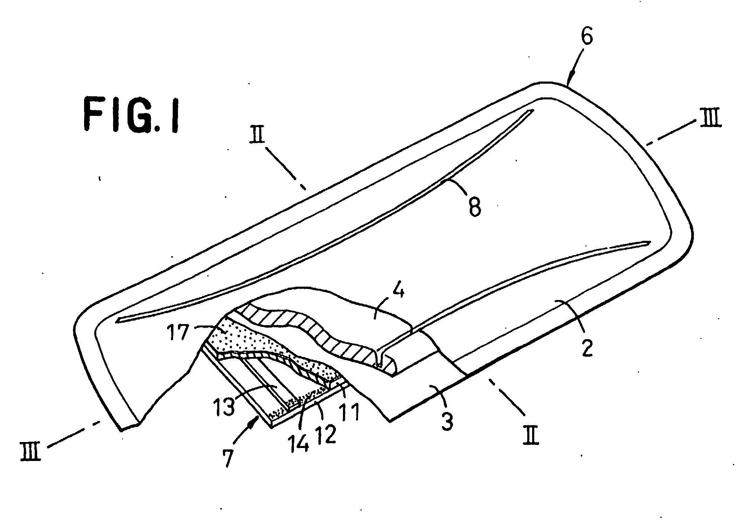

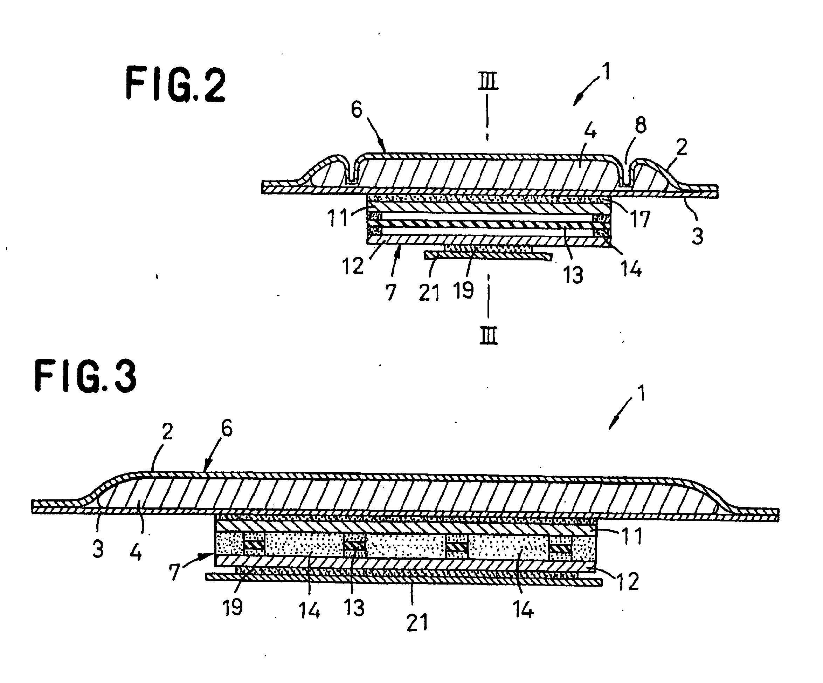

Fig. 1 is a perspective view showing a partially cut away sanitary napkin 1 and Figs. 2 and 3 are sectional views taken along lines II-II and III-III, respectively, in Fig. 1. The napkin 1 has a basic structure 6 including a liquid-pervious topsheet 2, a liquid-impervious backsheet 3 and a liquid-absorbent core 4 and deformable means 7 attached to the basic structure 6.

The basic structure 6 is configured to be elongate and has a transverse center line II-II dividing its length in two and a longitudinal center line III-III dividing its width in two. The topsheet 2 and the backsheet 3 extend outward beyond a peripheral edge of the absorbent core 4 so as to be placed upon and bonded to each other along their portions extending outside the peripheral edge of the absorbent core 4. An upper surface of the basic structure 6 is formed in the vicinity of its transversely opposite side edges with a pair of compressed grooves 8, respectively, describing curves which are convex toward the longitudinal center line III-III.

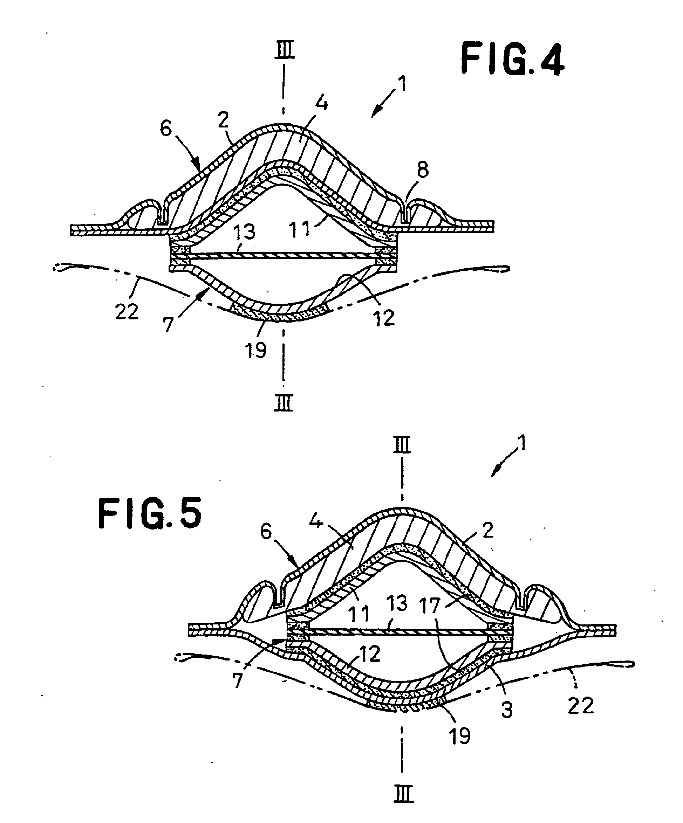

The deformable means 7 comprises upper and lower inelastic sheets 11, 12 placed upon each other and a plurality of ribbon-like elastic members 13 extending transversely of the basic structure 6 between the upper and lower sheets 11, 12. The upper and lower sheets 11, 12 are bonded by bonding means 14 such as hot melt adhesive to each other along their side edges. Each of the elastic members 13 has its longitudinally opposite ends being secured under tension to the side edges of the upper and lower sheets 11, 12 by the bonding means 14. The upper sheet 11 is bonded to a lower surface of the backsheet 3 by adhesive means 17 such as hot melt adhesive coating an upper surface of the upper sheet 11. The lower sheet 12 is coated on its lower surface along the longitudinal center line III-III with a layer of adhesive agent 19 which is protectively covered with a release sheet 21 (See Fig. 2). After peeling off the release sheet 21, the napkin 1 can be fastened to a crotch region of an undergarment 22 (See Fig. 4) worn by a napkin user by the adhesive agent 19.

Along each of the compressed grooves 8, the topsheet 2 is compressed together with the absorbent core 4 toward the backsheet 3 so that these sheets 2, 3 and absorbent core 4 may be substantially integrated. Consequently, the region of the absorbent core 4 compressed along each of the grooves 8 has a density higher than a density of the absorbent core 4 in its remaining region.

Fig. 4 is a sectional view taken along the transverse center line II-II, therein showing the napkin 1 fastened to the undergarment 22 indicated by an imaginary line and the elastic members 13 of the napkin 1 being in contracted state. The deformable means 7 presents an annular cross-section as the elastic members 13 having been placed under tension as shown in Fig. 2 contract as shown in Fig. 4. More specifically, contraction of the elastic members 13 causes the upper sheet 11 of the deformable means 7 to be deformed convexly upward and simultaneously causes the lower sheet 12 of the deformable means 7 to be deformed convexly downward. Deformation of the upper sheet 11 causes a region of the absorbent core 4 substantially defined between the pair of compressed grooves 8, 8 to be deformed convexly upward so that the region may be closely placed against a region around a napkin wearer's vaginal orifice with interposition of the topsheet 2. The wearer's body weight exerted upon the napkin 1 in the state as seen in Fig. 4 will cause the elastic members 13 to be stretched again and thereby cause the napkin 1 to be flattened again as seen in Fig. 2. Stretch and contract of the elastic members 13 are not obstructed by fastening of the deformable means 7 to the undergarment 22 because the deformable means 7 is fastened to the undergarment 22 only along the longitudinal center line III-III and in the vicinity thereof.

The upper and lower sheets 11, 12 operating in such a manner in the deformable means 7 occupy 50 - 100 % of a width of the absorbent core 4 in the direction of the transverse center line II-II and 10 - 80 % of a length of the absorbent core 4 in the direction of the longitudinal center line III-III. The adhesive agent 19 is formed about the longitudinal center line III-III and has a width in the direction of the transverse center line II-II corresponds to 10 - 50 % of the width of the absorbent core 4 so that the side edges of the lower sheet 12 and a region in the vicinity of these side edges may be left not fastened to the undergarment 22. Transversely of the absorbent core 4, both the upper sheet 11 and the lower sheet 12 preferably have rigidities corresponding to or higher than a rigidity of the absorbent core 4. It should be understood, in this regard, that both the upper sheet 11 and the lower sheet 12 may have relatively lower rigidities along the longitudinal center line III-III and a region in the vicinity thereof in order to facilitate flection of these sheets 11, 12 along the longitudinal center line III-III.

Fig. 5 is a view similar to Fig. 4 showing another embodiment of this invention. The napkin 1 according to this embodiment is similar to the previous embodiment shown in Fig. 4 except that the deformable means 7 shown in Fig. 4 is disposed between the absorbent core 4 and the backsheet 3 and bonded to these absorbent core 4 and backsheet 3, respectively, by layers of adhesive agent 17. The backsheet 3 is coated on its lower surface with a layer of adhesive agent 19 extending along the longitudinal center line III-III so that the napkin 1 may be fastened to an inner surface of the undergarment 22 by the adhesive agent 19. Contraction of the elastic members 13 causes the topsheet 2, the absorbent core 4 and the upper sheet 11 to be deformed convexly upward and simultaneously causes the backsheet 3 and the lower sheet 12 to be deformed convexly downward. Regarding the range over which the backsheet 3 is coated with the layer of adhesive agent 19, this embodiment is similar to the previous embodiment shown in Fig. 4.

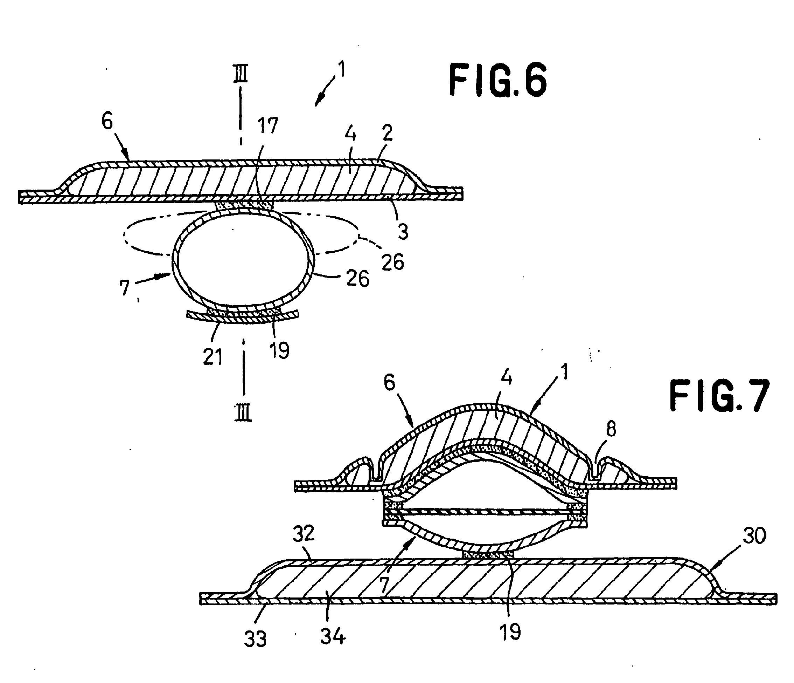

Fig. 6 is also a view similar to Fig. 4 showing still another embodiment of this invention. The sanitary napkin 1 according to this embodiment is distinguished from the previous embodiment in that the deformable means 7 adopted by this embodiment has an arrangement differing from the arrangement as shown in Fig. 4 and the napkin basic structure 6 has no compressed grooves 8 formed thereon. The deformable means 7 specifically comprises a member 26 which is made from an elastic sheet material such as a rubber- or elastomer-sheet and presents an annular shape as viewed in a section taken transversely of the napkin 1. The member 26 extends along the longitudinal center line III-III of the napkin 1 and is secured over a region extending along the longitudinal center line III-III and in the vicinity thereof to the lower surface of the backsheet 3 by a layer of adhesive agent 17. The member 26 is coated over a region diametrically opposite to the region of bonding with a layer of adhesive agent 19 which is protectively covered with a release sheet 21. A wearer's body weight exerted on the napkin 1 from above causes the annular member 26 to be depressed and thereby to be elastically flattened as indicated by an imaginary line.

Fig. 7 is a view similar to Fig. 4 illustrating a manner in which the napkin shown in Fig. 1 is possibly used in combination with an additional napkin. As shown, the napkin 1 overlies a second napkin 30 having a width larger than that of the napkin 1 so as to lie in the transversely middle zone of the second napkin 30. The second napkin 30 comprises a liquid-pervious topsheet 32, a liquid-impervious backsheet 33 and a liquid-absorbent core 34 disposed between these two sheets 32, 33. The napkin 1 is bonded to an upper surface of the topsheet 32 by a layer of adhesive agent 19. In order to ensure that, in the napkin 1, the absorbent core 4 can adequately follow a movement of the deformable means 7, the absorbent core 4 must be relatively thin. If it is apprehended, in this case, that the absorbent core 4 might be too thin to meet an absorbing capacity desired for the absorbent core 4, the napkin 1 may be used in combination with the additional napkin 30 as shown in Fig. 7 to avoid such apprehension and simultaneously to improve fitting of the napkin 1 to the region around wearer's vaginal orifice.

It is possible without departing from the scope of this invention to replace a plurality of elastic members 13 by a single elastic member 13. It is also possible to replace the plurality of elastic members 13 by a single elastic sheet having a same size as the upper and lower sheets 11, 12. The compressed grooves 8 not only serve to define the lines along which the napkin 1 starts to protrude upward but also serve to receive a partial amount of menstrual discharge flowing sideways along the sloped topsheet 2 to prevent such amount of menstrual discharge from leaking sideways. The curved grooves 8 as in the illustrated embodiment may be replaced by rectilinear grooves 8. It is also possible without departing the scope of this invention to eliminate the grooves 8 at all.

For exploitation of this invention, two or more members may be bonded together using suitable adhesive agent such as hot melt adhesive agent or, depending on the nature of the members, using heat-sealing or ultrasonic sealing technique.

The sanitary napkin according to this invention is allowed to maintain its proper fitting to the region around a wearer's vaginal orifice without relying upon a pressure exerted by the wearer's femora on the napkin. Such advantageous effect is achieved by the unique arrangement according to this invention that the liquid-absorbent core is deformed convexly upward by the elastically deformable means and the napkin is fastened only along its transversely middle region to the undergarment worn by the wearer in order to assure unobstructed deformation of the absorbent core.

## Claims

1. A sanitary napkin (1) having a basic structure (6) including a liquid-pervious topsheet (2) for defining a body-facing side, a liquid-impervious backsheet (3) for defining an undergarment-facing side and a liquid-absorbent core (4) disposed between said topsheet (2) and said backsheet (3), and deformable means (7) adapted to deform a transversely middle region of said absorbent core (4) convexly toward the body-facing side of said napkin (1) and fastening means (19) used to detachably fasten said napkin (1) to an undergarment,
**characterised in that**
said deformable means (7) includes either an elastic sheet (26) presenting an annular shape transversely of said absorbent core (4) or paired inelastic sheet-like members (11, 12) of the same width placed upon each other, and an elastically stretchable/contractible member (13) extending under tension between said paired sheet-like members (11, 12) transversely of said absorbent core (4) and fixed to transversely opposite side edges of said paired sheet-like members (11, 12),
said fastening means (19) extends on a lower surface of said napkin along a longitudinal center line and has a width corresponding to 5 - 50 % of a width of said absorbent core (2).

2. A sanitary napkin according to Claim 1, wherein said napkin is bonded by said fastening means (19) to an upper surface of a second napkin (30) contoured to be larger than said napkin (1).

## Revendications

1. Serviette hygiénique (1) comportant une structure de base (6) comprenant une feuille de dessus (2) perméable aux liquides destinée à définir un côté faisant face au corps, une couche postérieure (3) imperméable aux liquides destinée à définir un côté faisant face à un sous-vêtement et un coeur (4) absorbant les liquides disposé entre ladite couche de dessus (2) et ladite couche postérieure (3), et un moyen déformable (7) adapté à déformer une région transversalement médiane dudit coeur absorbant (4) de manière convexe vers le côté faisant face au corps de ladite serviette (1), ainsi qu'un moyen de fixation (19) utilisé pour fixer de manière détachable ladite serviette (1) à un sous-vêtement, **caractérisée en ce que** :
ledit moyen déformable (7) comprend soit une feuille élastique (26) présentant une forme annulaire transversalement audit coeur absorbant (4), soit une paire d'éléments non élastiques de type feuille (11, 12), de même largeur, placés l'un au-dessus de l'autre, et un élément (13) élastiquement étirable/contractable s'étendant sous tension entre les éléments de type feuille (11, 12) de ladite paire transversalement audit coeur absorbant (4) et fixé aux bords latéraux transversalement opposés des éléments de type feuille (11, 12) de ladite paire,
ledit moyen de fixation (19) s'étend sur une surface inférieure de ladite serviette selon un axe médian longitudinal, et a une largeur correspondant à 5 à 50 % de la largeur dudit coeur absorbant (4).

2. Serviette hygiénique selon la revendication 1, dans laquelle ladite serviette est liée par ledit moyen de fixation (19) à la surface supérieure d'une deuxième serviette hygiénique (30) dont le contour est plus grand que ladite serviette (1).

## Patentansprüche

1. Damenbinde (1) mit einer Grundstruktur (6), die eine flüssigkeitsdurchlässige obere Lage (2) zur Bildung einer körperzugewandten Seite, eine flüssigkeitsundurchlässige rückwärtige Lage (3) zur Bildung einer Unterbekleidungs-zugewandten Seite und einen zwischen der oberen Lage (2) und der rückwärtigen Lage (3) angeordneten flüssigkeitsabsorbierenden Kern (4) enthält, und mit einer verformbaren Einrichtung (7) zur Verformung eines in Querrichtung mittleren Bereichs des absorbierenden Kerns (4) konvex zur körperzugewandten Seite der Binde (1) hin sowie einer Befestigungseinrichtung (19) zum lösbaren Befestigen der Binde (1) an der Unterbekleidung,
**dadurch gekennzeichnet, daß**
die verformbare Einrichtung (7) entweder eine elastische Lage (26), die quer zum absorbierenden Kern (4) eine Ringform zeigt, oder paarweise vorgesehene inelastische lagenartige Elemente (11, 12) gleicher Breite, die aufeinander angeordnet sind, sowie ein elastisch dehnbares bzw. zusammenziehbares Element (13), das sich unter Spannung zwischen den paarweise angeordneten lagenartigen Elementen (11, 12) quer zum absorbierenden Kern (4) erstreckt und an einander in Querrichtung gegenüberliegenden Seitenkanten der paarweise angeordneten lagenartigen Elemente (11, 12) befestigt ist, enthält, und
die Befestigungseinrichtung (19) auf einer unteren Fläche der Binde entlang einer Längsmittellinie verläuft und eine Breite aufweist, die 5 bis 50 % der Breite des absorbierenden Kerns (2) entspricht.

2. Damenbinde nach Anspruch 1, die mittels der Befestigungseinrichtung (19) an eine zweite Binde (30) gebondet ist, die einen größeren Umriß als die genannte Binde (1) aufweist.
